# DEMANDE DE BREVET EUROPEEN

(11) **EP 2 857 494 A1**
(43) Date de publication de la demande: **08.04.2015**
(21) Numéro de dépôt: 14186981.8
(22) Date de dépôt: 30.09.2014
(51) Int. Cl.: C12M 1/107, C12M 3/00, C12M 1/00

(54) **Installation de digestion anaérobie pour effluents aptes à subir un procédé de méthanisation en phase fluide**

(30) Priorité: 01.10.2013 FR 1359502
(71) Demandeur: Janvier, Regis, 22170 Plerneuf (FR)
(72) Inventeur: Janvier, Regis, 22170 Plerneuf (FR)
(74) Mandataire: Maillet, Alain

(57) **Abrégé**

La présente invention se situe dans le domaine technique du traitement de la digestion anaérobie et concerne une installation de traitement des effluents aptes à subir une méthanisation par voie liquide, ou fluide.

L'installation de digestion (100) selon l'invention se caractérise en ce qu'elle comprend une pluralité de modules de digestion (101, 102, 103) reliés entre eux par un moyen de circulation (104) de la phase liquide de l'effluent à traiter.

## Description

La présente invention se situe dans le domaine technique du traitement de la digestion anaérobie et concerne une installation de traitement des effluents aptes à subir une méthanisation par voie liquide, ou fluide.

La méthanisation, ou digestion anaérobie, est la décomposition par des microorganismes, en l'absence d'oxygène, de la matière organique contenue dans les effluents à traiter. Cette décomposition de la matière organique aboutit au dégagement de gaz, principalement du méthane.

La digestion anaérobie par voie fluide des effluents, c'est à dire des effluents contenant au maximum 25% de matière sèche, tels que des boues de stations d'épuration, des effluents industriels ou agricoles, des fumiers ou certain déchets végétaux ou organiques, se déroule soit à l'intérieur de grands réservoirs circulaires en acier vitrifié ou en béton hermétiquement clos et à l'intérieur desquels sont prévus des systèmes d'agitation ou de brassage du substrat et des systèmes de régulation de la température, soit dans des réacteurs de type à piston, selon un principe d'agitation mécanique selon un axe longitudinal et par avancement continu de la matière.

Les dispositifs actuellement proposés nécessitent tous la mise en oeuvre de génie civil important, avec, *a minima,* l'installation d'une dalle de béton sur le site d'installation pour l'accueil de ceux ci. Lorsque l'installation a vocation à traiter de plus grandes quantités d'effluents, elle doit être remplacée par une installation de plus grande taille ou complétée par l'adjonction de cuves supplémentaires autonomes.

Le but de la présente invention est de proposer une installation de digestion anaérobie pour effluents aptes à subir un procédé de méthanisation en phase fluide qui soit standard, simple et rapide à installer, et qui présente notamment une isolation thermique optimale sans ponts thermiques, une bonne protection contre la corrosion, une conception permettant le montage et le démontage en quelques heures.

L'installation selon l'invention doit pouvoir, en outre, être augmentée facilement et rapidement en taille de manière à traiter des volumes d'effluents plus conséquents, au besoin.

A cet effet, l'invention concerne une installation de digestion anaérobie pour effluents aptes à subir un procédé de méthanisation en phase fluide, qui se caractérise en ce qu'elle comprend une pluralité de modules de digestion reliés entre eux par un moyen de circulation interne de la phase liquide de l'effluent à traiter.

Les modules de l'installation sont montés en série et communiquent donc entre eux par un moyen de circulation interne de la phase liquide de l'effluent.

La circulation interne de la phase liquide entre les modules se produit à une vitesse différente de celle de la phase solide, à la différence de ce qui se produit dans un digesteur de type classique à piston.

Préférentiellement, le moyen de circulation est un moyen de circulation par gravité, par exemple, par déversement d'un module à l'autre. Ceci peut être réalisé à l'aide de conduits, de cannes de jonction ou de trappes communiquant d'un module à l'autre.

Avantageusement, chaque module est composé d'une cuve de forme parallélépipédique ou cubique. Les dimensions choisies seront préférentiellement telles que chaque module sera transportable par la route.

Selon un mode de réalisation de l'invention, les modules de digestion sont disposés en série, la paroi d'un premier module apposée contre la paroi du module suivant. Cette caractéristique permet de proposer une installation compacte et permet de limiter l'encombrement au sol.

De plus, selon ce mode de réalisation, les cuves, posées côte à côte et communiquant par le moyen de circulation de phase liquide, ont toujours le même niveau de remplissage de liquide. Ainsi, les parois mitoyennes reçoivent des poussées identiques et qui s'annulent de part et d'autre. Cela participe à renforcer la résistance mécanique de l'ensemble puisque les parois d'un module sont consolidées par la paroi du module adjacent.

Préférentiellement, chaque module comprend un moyen d'agitation de l'effluent à traiter.

Préférentiellement, il s'agit d'un moyen d'agitation par injection de gaz. Un tel moyen est bien connu en tant que tel et se présente sous la forme d'un conduit en fond de cuve comportant des perforations par lesquelles s'échappe un gaz. Les remontées de bulles de gaz permettent une agitation de la matière à l'intérieur de la cuve.

Plus préférentiellement, le moyen d'agitation par injection de gaz est un dispositif de circulation de gaz commun à l'ensemble des modules. Ainsi, un seul moteur externe permet l'agitation de plusieurs modules.

Préférentiellement, chaque module comprend au moins un moyen de fixation à un module adjacent. Préférentiellement, il s'agit d'un ensemble de brides de raccord.

Préférentiellement, chaque module comprend un moyen de raccordement à un conduit de collecte du gaz issu du procédé de méthanisation. En effet, l'installation comprend de manière classique un système de récupération du biogaz produit. Chaque module est connecté à ce système.

Avantageusement, chaque module comporte un fond constitué par une dalle de béton, ou par une dalle métallique, solidaire du module. Cette disposition constructive permet de poser directement le module sur le site de telle sorte qu'une simple plate-forme de terre stabilisée suffit sans qu'il ne soit nécessaire de prévoir une dalle béton. D'autre part cette épaisseur de dalle est de l'ordre d'une dizaine de centimètres dans le cas d'une dalle de béton, ou quelques millimètres dans le cas de l'acier, et assure par la suite la stabilité dimensionnelle de l'ensemble et assurant la planéité du sol, ce qui lui permettra de supporter une charge de liquide de plus de 3 tonnes par mètre carré sans déformation.

Selon l'invention, chaque module est muni d'un revêtement intérieur contre la corrosion, tel qu'une peinture bitumineuse, époxy ou similaire.

Avantageusement, l'installation comporte au moins une membrane souple de type EPDM (éthylène-propylène-diène monomère) ou PVC prévue pour recouvrir la partie supérieure de chaque module ou de l'ensemble des modules. La membrane souple permet de protéger le métal de la corrosion par le gaz.

Avantageusement encore, chaque module comporte une toiture, par exemple en acier, et munie d'une trappe souple composée d'une membrane extensible. La fonction de cette membrane extensible est d'absorber les augmentations de pression liées à l'injection de gaz pour l'agitation. La trappe pourra avantageusement représenter la totalité de la toiture.

Avantageusement, le dessus des modules forme une plate-forme sur laquelle est fixée une poche fermée par une membrane en matériau EPDM permettant le stockage du biogaz.

Avantageusement, l'installation comporte un revêtement isolant par l'extérieur, commun à la pluralité de modules. Les modules étant accolés les uns à la suite des autres dans un montage en série, l'installation est compacte et permet la mise en oeuvre d'un revêtement en matériau isolant commun. Cette caractéristique limite les pertes thermiques par rapport à la mise en oeuvre d'une isolation de chaque module séparément. A titre d'exemple, l'ensemble formé par les modules pourra être isolé par des plaques préfabriquées comportant un matériau isolant et une structure externe en bois.

Avantageusement, chaque module comporte un système de chauffage ou tout autre dispositif utilisé dans un tel digesteur et connu pour permettre le bon déroulement de la méthanisation. L'homme du métier connaît bien les dispositifs utilisés habituellement dans ce type de digesteur, tels que les dispositifs d'agitation et de chauffage, et saura sans difficultés les adapter à une installation selon l'invention.

Selon un mode de réalisation de l'invention, l'installation comprend un moyen de recirculation de l'effluent liquide depuis le dernier module de la série jusqu'au premier. L'effluent liquide circule en effet de modules en modules, d'un premier module jusqu'à un dernier module. Le moyen de recirculation permet de réacheminer l'effluent liquide de dernier module jusque dans le premier module de manière à créer une boucle de circulation.

Selon un mode de réalisation de l'invention, les modules sont posés directement sur le sol non bétonné et recouvert de plaques de matériaux isolants non durs et résistants à la compression, type polystyrène ou équivalent permettant d'isoler les fonds des modules du sol, tant sur le plan thermique que des remontées d'humidité. Ces plaques assurent également la planéité finale de la surface.

Selon un mode de réalisation, les modules sont munis d'une face supérieure amovible permettant le nettoyage de la cuve.

Selon un autre mode de réalisation, l'installation comprend, en amont des modules de digestion, une cuve comprenant un dispositif de mélange de l'effluent à méthaniser. Cette cuve permet d'homogénéiser correctement l'effluent préalablement à son introduction dans les modules de digestion.

Préférentiellement, la cuve de mélange présente une longueur et une largeur telle que la longueur est comprise entre 2,5 fois et 5,5 fois la largeur.

Préférentiellement encore, le dispositif de mélange est une hélice disposée dans le sens de la longueur.

Préférentiellement encore, la cuve de mélange repose sur des pesons, permettant ainsi de mesurer les quantités entrant dans le mélange, ainsi que les quantités alimentant les modules de digestion.

Les caractéristiques de l'invention, ainsi que d'autres, apparaîtront plus clairement à la lecture des modes de réalisation suivants :
La Fig. 1 est une représentation schématique d'une installation selon l'invention,
La Fig. 2 est une représentation schématique d'un module de digestion en coupe d'une installation selon l'invention,
La Fig. 3 est une représentation schématique en perspective d'un module de digestion d'une installation selon l'invention,
La Fig. 4 est une représentation schématique en perspective d'une installation selon l'invention comportant trois modules de digestion,
La Fig. 5 est une représentation schématique d'une cuve de pré mélange d'une installation selon l'invention, et
La Fig. 6 est une représentation schématique en coupe de trois modules de digestion d'une installation selon l'invention.

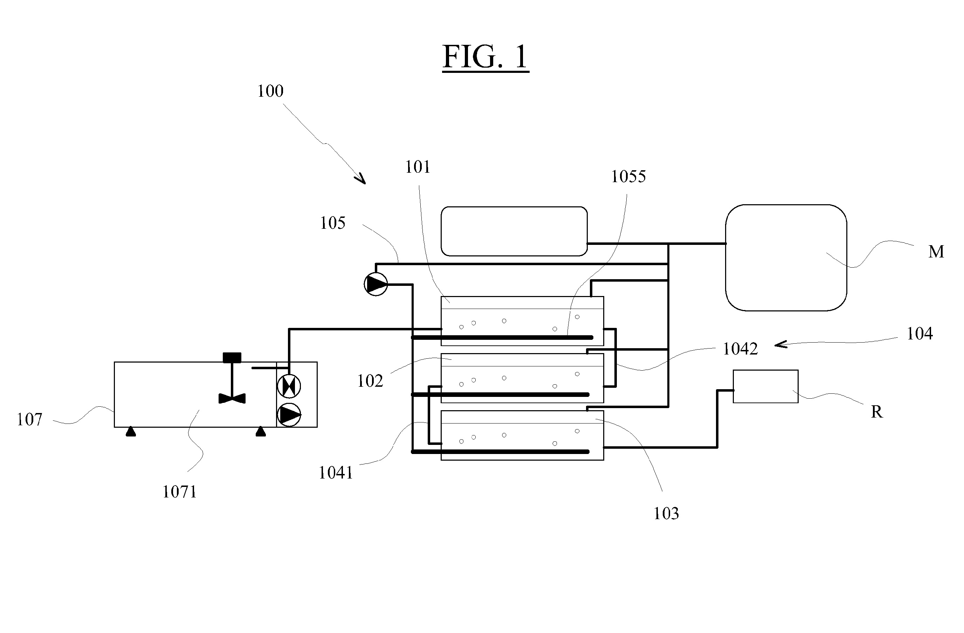

En lien avec la Fig. 1 est représentée une installation de digestion anaérobie pour effluents aptes à subir un procédé de méthanisation en phase fluide 100.

L'installation 100 comporte, dans cet exemple, trois modules de digestion 101, 102, 103 montés en série et à l'intérieur desquels se déroule la méthanisation.

L'installation 100 comporte encore un moyen de circulation 104 de l'effluent liquide d'un module au module adjacent. Dans ce mode de réalisation, le moyen de circulation 104 est un moyen de circulation par gravité, réalisé par exemple, par des conduits 1041 et 1042 réunissant les modules adjacents entre eux et qui permet le déversement du liquide d'un module à l'autre par gravité afin de répartir un volume égal de l'effluent liquide d'un module à l'autre.

L'installation comporte encore une cuve de mélange de l'effluent 107 préalablement à son introduction dans les modules de digestion 102, 102 ,103.

L'effluent à traiter est acheminé depuis la cuve de mélange 107 jusqu'au premier module de digestion 101. Le module 101 est relié au module 102 par le conduit 1041 permettant la circulation de la phase liquide de l'effluent à traiter du module 101 au module 102. De même, le module 102 est relié au module 103 par le conduit 1042 permettant la circulation de la phase liquide de l'effluent à traiter du module 102 au module 103. L'effluent traité est enfin acheminé vers un conteneur de récupération R.

Les cuves 101, 102, 103 comportent donc la même quantité de liquide. Ainsi, les parois mitoyennes reçoivent des poussées qui s'annulent de part et d'autre. Cela participe à renforcer la résistance mécanique de l'ensemble puisque les parois d'un module sont consolidées par la paroi du module adjacent.

L'installation comporte encore un dispositif de circulation du gaz issu de la méthanisation de l'effluent 105. Le dispositif 105 est composé d'un réseau de conduits qui récupèrent le gaz produit et de conduits 1051 qui pénètrent à l'intérieur de chaque module de digestion 101, 102, 103. Ces conduits 1051 sont percés et laissent échapper des bulles de gaz à l'intérieur de chaque module de digestion. Le mouvement des bulles de gaz produit l'agitation. Le dispositif 105 forme donc un moyen d'agitation 1055 de l'effluent liquide.

Le dispositif 105 est en outre alimenté en énergie par un moteur unique M.

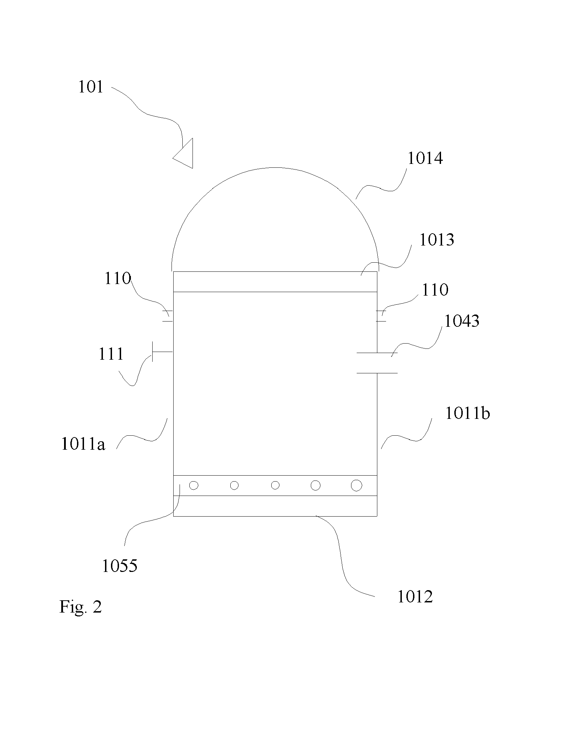

Un module de digestion 101 est représenté sur la Fig. 2. Le module 101 est une cuve aux parois latérales 1011a et 1011b réalisées en acier. Elle comporte une dalle métallique, par exemple en acier, formant un fond 1012 et un plafond en acier 1013. Le module 101 est recouvert en partie supérieure d'une membrane souple en EPDM 1014.

Un moyen de raccordement 1043 au moyen de circulation de l'effluent liquide 104 permettant le déversement de la cuve 101 vers une autre cuve est prévu. Le moyen de raccordement 1041 est par exemple une trappe ou fenêtre pratiquée dans la paroi latérale 1011a ou 101b, ou encore un conduit.

Le module 101 comporte des moyens de raccordement 110 à un module suivant, tels que des brides de serrage ainsi qu'un moyen de raccordement 111 au dispositif de circulation de gaz 105.

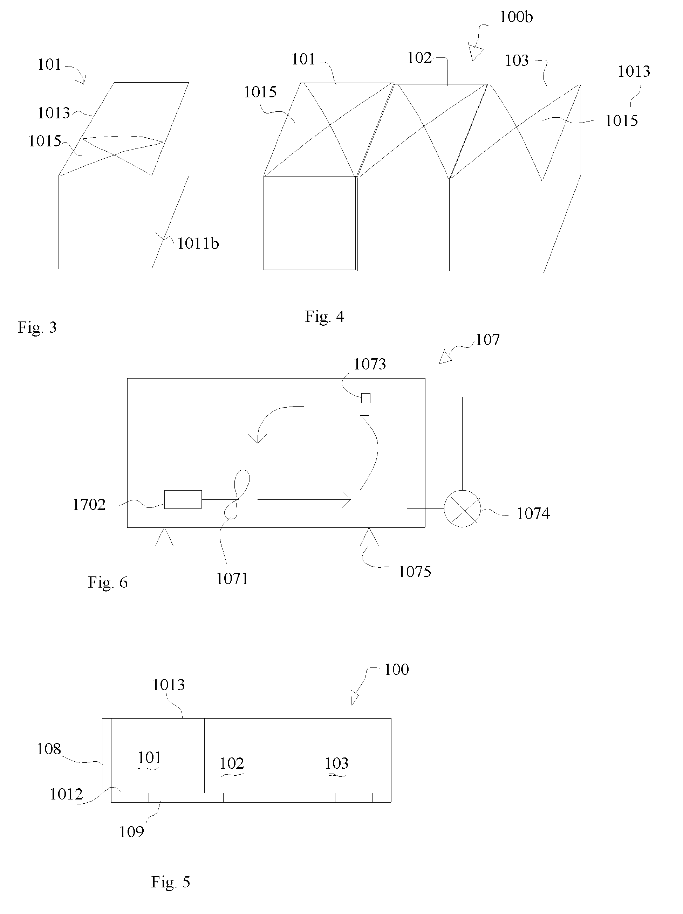

Le plafond 1013 présente une trappe 1015 réalisée en un matériau souple et élastique (Fig. 3). La trappe permet d'absorber les variations de pressions à l'intérieur de la cuve.

A la Fig. 4 sont représentés des modules de digestion 101, 102 et 103 dont les plafonds 1103, 1113 et 1123 sont, dans ce mode de réalisation, entièrement constitués d'une trappe 1015 en matériau souple et élastique.

L'ensemble des modules 101, 102 et 103 est isolé thermiquement par un revêtement externe 108 (Fig. 5) se présentant par exemple sous forme de plaques à assembler. Tout matériau isolant connu peut être utilisé. L'installation repose sur des dalles 109 de matériau isolant non dur, type polystyrène ou équivalent.

La cuve de mélange de l'effluent 107 est illustrée plus en détail à la Fig. 6. Elle comporte une hélice 1071, mise en mouvement par un moteur immergé 1072. L'hélice 1071 présente un diamètre compris entre 25% et 50 % de la largeur de la cuve. Cette hélice est positionnée avantageusement dans la moitié inférieure de la cuve de mélange 107 à une distance comprise entre 1/6 et 1/12 de la longueur du bord de la cuve. La vitesse de rotation de l'hélice et la puissance sont telles que le débit induit est supérieur à 4 fois le volume de la cuve par heure. L'hélice 1071 induit un mouvement longitudinal poussant en fond de cuve et un retour se crée alors en partie supérieure de la cuve, ainsi qu'un vortex d'aspiration au droit de l'hélice. Le mouvement de retour en partie supérieure de la cuve sera favorisé par la disposition d'un éjecteur 1073 poussant dans le sens opposé à l'hélice. L'éjecteur pourra être alimenté par une pompe 1074 immergée ou non. D'autre part le flux horaire passant dans l'éjecteur 1073 et ayant transité par la pompe 1074 représentera entre 1 fois et 4 fois le volume de la cuve 107. La pompe 1074 pourra être munie d'un principe de dilacération ou de broyage. Cette disposition oblige les matières cellulosiques, par exemple de la paille, à se rassembler dans la partie haute au dessus de l'hélice 1071 où elles seront happées par le vortex induit par la rotation de celle ci. D'autre part, la disposition décrite ci dessus permet également un passage de 100% du volume dans l'hélice dans un temps réduit et de manière fiable. La cuve est maintenue à une température supérieure à 42° C et inférieure à 50° C, afin de permettre le développement de bactéries permettant l'hydrolyse, sans pour autant permettre la production de méthane.

## Revendications

1. Installation de digestion (100) anaérobie pour effluents aptes à subir un procédé de méthanisation en phase fluide, **caractérisée en ce qu'**elle comprend une pluralité de modules de digestion (101, 102, 103), la paroi d'un module étant apposée contre la paroi du module suivant, les modules étant reliés entre eux par un moyen de circulation (104) de la phase liquide de l'effluent à traiter par gravité d'un module à l'autre afin de répartir un volume égal d'effluent liquide d'un module à l'autre.

2. Installation de digestion (100) selon la revendication 1, **caractérisée en ce que** les modules de digestion sont disposés en série, la paroi d'un premier module (101) apposée contre la paroi du module suivant (102).

3. Installation de digestion (100) selon l'une des revendications précédentes, **caractérisée en ce que** chaque module de digestion (101, 102, 103) est composé d'une cuve de forme parallélépipédique ou cubique.

4. Installation de digestion (100) selon l'une des revendications précédentes, **caractérisée en ce qu'**elle comprend un moyen d'agitation (1055) de l'effluent à traiter commun à l'ensemble desdits modules.

5. Installation de digestion (100) selon l'une des revendications précédentes, **caractérisée en ce que** chaque module de digestion (101, 102, 103) comprend au moins un moyen de fixation (106) à un module adjacent.

6. Installation de digestion (100) selon l'une des revendications précédentes, **caractérisée en ce que** chaque module de digestion (101, 102, 103) comprend au moins un moyen de raccordement (111) à un dispositif de circulation (105) du gaz issu du procédé de méthanisation.

7. Installation de digestion (100) selon l'une des revendications précédentes, **caractérisée en ce que** chaque module de digestion (101, 102, 103) comporte un fond (1012) constitué par une dalle de béton, ou par une dalle métallique, solidaire du module.

8. Installation de digestion (100) selon l'une des revendications précédentes, **caractérisée en ce que** chaque module (101, 102, 103) comporte un plafond (1013), par exemple en acier, muni d'une trappe (1015) souple composée d'une membrane extensible.

9. Installation de digestion (100) selon l'une des revendications précédentes, **caractérisée en ce qu'**elle comporte un revêtement isolant par l'extérieur (108), commun à la pluralité de modules (101, 102, 103).

10. Installation de digestion (100) selon l'une des revendications précédentes, **caractérisée en ce qu'**elle comporte, en amont des modules de digestion (101, 102, 103), une cuve de mélange (107) comprenant un dispositif de mélange (1071) de l'effluent à méthaniser.

11. Installation de digestion (100) selon l'une des revendications précédentes, **caractérisée en ce que** la cuve de mélange (107) présente une longueur et une largeur telle que la longueur est comprise entre 2,5 fois et 5,5 fois la largeur.

12. Installation de digestion (100) selon l'une des revendications précédentes, **caractérisée en ce que** la cuve de mélange (107) repose sur des pesons (1075).

13. Installation de digestion (100) selon l'une des revendications précédentes, **caractérisée en ce que** les modules de digestion (101, 102, 103) reposent sur un sol non bétonné et recouvert de dalles en matériau isolant thermiquement et assurant la planéité du sol.

14. Module de digestion (101, 102, 103) pour la digestion anaérobie d'effluents aptes à subir un procédé de méthanisation en phase fluide, **caractérisé en ce qu'**il comprend au moins un moyen de fixation (106) à un module adjacent ainsi qu'un moyen de raccordement (1043) à un moyen de circulation (104) d'effluent liquide d'une installation de méthanisation (100) telle que définie dans l'une des revendications 1 à 13.
